# EUROPÄISCHE PATENTANMELDUNG

(11) **EP 1 955 733 A1**
(43) Veröffentlichungstag der Anmeldung: **13.08.2008**
(21) Anmeldenummer: 07002475.7
(22) Anmeldetag: 06.02.2007
(51) Int. Cl.: A61N 5/10

(54) **Vorrichtung zur räumlichen Lokalisation eines bewegbaren Körperteils**

(71) Anmelder: Siemens Schweiz AG, 8047 Zürich (CH)
(72) Erfinder: Broennimann, Thomas, 4704 Wolfisberg (CH); Cloutot, Laurent, 8956 Killwangen (CH)
(74) Vertreter: Fischer, Michael

(57) **Zusammenfassung**

Es wird eine Vorrichtung zur räumlichen Lokalisation bzw. zur räumlichen Verfolgung eines innerhalb eines Bewegungsvolumens bewegbaren Körperteils vorgeschlagen, welcher eine in dem Bewegungsvolumen eingeschränkte Bewegungsbahn an der Oberfläche oder/und auch in Innerem eines lebenden Wesens aufweist. Die Vorrichtung weist folgende Komponenten auf:
- eine Schallempfängervorrichtung, welche außerhalb des Wesens angeordnet ist,
- eine Wellenquelle wie eine Schallquelle, welche außerhalb des Wesens angeordnet ist und aus welcher sich mindestens eine erste Welle, vorzugsweise eine Schallwelle, bis zum Bewegungsvolumen ausbreitet,
- die erste Schallwelle einen spektralen Erreger auftrifft, welcher dadurch in Schwingung versetzt wird und dessen zum Körperteil relative Lage bekannt ist und aus welchem aufgrund der Schwingung mindestens eine zweite, ausgehende Schallwelle emittiert wird, welche sich weiterhin bis zur Schallempfängervorrichtung ausbreitet und an der Schallempfängervorrichtung isolierbar ist,
- die Schallempfängervorrichtung eine Mehrzahl von räumlich unterscheidbaren (d.h getrennten oder zeitlich versetzbaren) Schallempfängereingängen aufweist, welche in einem bekannten Koordinatensystem derart gelagert sind, dass die zweite Schallwelle zumindest eine maximale Zahl von Schallempfängereingängen eintrifft.

## Beschreibung

Die Erfindung betrifft eine Vorrichtung zur räumlichen Lokalisation eines bewegbaren Körperteils nach dem Oberbegriff des Anspruches 1.

Bei einer Bestrahlungstherapie eines Tumors z.B. mittels einer Gamma- oder Protonenbestrahlung, oder bei einer Abbildung eines Tumors im Inneren des Körpers eines Wesens existieren zahlreiche Methoden oder Vorrichtungen, bei welchen es angestrebt wird, eine möglichst genaue und reproduzierbare dreidimensionale Lokalisation des Tumors oder allgemeiner eines bewegbaren Körperteils zu gewährleisten. Einige Methoden ("in-vivo"-Abbildungsmethoden z.B. mittels einer Radiographie mit X-Strahlung) sind aber als schädliche ("invasive") Methoden eingestuft und sollten dadurch nur in eingeschränkter Weise verwendet werden. Auch weniger schädliche "in-vivo"-Methoden, wie z.B. eine Abbildungsmethode mittels magnetischer Resonanz, bleiben zusätzlich zu ihrer Komplexität, ihrem Aufwand und ihren Kosten mit und gleichzeitig zu einer Bestrahlungsbehandlung schwer kombinierbar. Andere Methoden ("ex-vivo") nutzen (z.B. optisch) detektierbare Merkmale/Markierungen, welche an einem Patient relativ zu einem bekannten Koordinatensystem anbringbar oder/und erkennbar sind. Jedoch leiden diese Methoden an Genauigkeit und sowieso an Reproduzierbarkeit, falls physiologische Änderungen des Patienten (z.B. Gewichtabnahme) oder unvermeidliche (am schlimmsten unerwartete) Bewegungen (z.B. durch den Atem) während Behandlungen bzw. Neupositionierungen des Patienten/Körperteilen zwischen Behandlungen auftreten. Es existieren ferner Verfahren/Vorrichtungen zur Festhaltung des Patienten oder eines Körperteils an z.B. einem Liegetisch bei der Bestrahlungstherapie oder Messeinrichtungen des Atems, jedoch verringern diese Lösungen einerseits den "Komfort" des Patienten bzw. ermöglichen keine 100%-genaue räumliche Festlegung/Lokalisation eines Tumors in einem bekannten Koordinaten-system.

Der Erfindung liegt eine Aufgabe zugrunde, eine Vorrichtung zur zuverlässigen, räumlichen (d.h. ein-, zwei oder dreidimensionalen) Lokalisation (d.h. auch zur genauen, räumlich dynamischen Verfolgung) eines bewegbaren Körperteils (Tumor, Karzinom, etc) eines lebenden Wesens (d.h. auch eines menschlichen Patienten) anzugeben.

Ferner sollte dafür eine potentielle Gefahr mit belastenden (= invasiven) Strahlungen des Patienten vermieden werden und insbesondere sollte die Vorrichtung gleichzeitig während einer Gamma-Bestrahlungstherapie eines Patienten z.B. zur Vernichtung eines Tumors oder eines Karzinoms möglichst in Echtzeit und den Körperteil "in-vivo" möglichst dreidimensional über genaue, absolute Koordinaten in einem bekannten Koordinatensystem lokalisieren können.

Erfindungsgemäß wird die Aufgabe durch die Merkmale des Anspruches 1 gelöst.

Es wird eine Vorrichtung zur räumlichen Lokalisation bzw. zur räumlichen Verfolgung eines innerhalb eines Bewegungsvolwmens bewegbaren Körperteils vorgeschlagen, welcher eine in dem Bewegungsvolumen eingeschränkte Bewegungsbahn an der Oberfläche oder/und auch in Innerem eines lebenden Wesens aufweist. Die Vorrichtung weist folgende Komponenten auf:
- eine Schallempfängervorrichtung, welche außerhalb des Wesens angeordnet ist,
- eine Wellenquelle wie eine Schallquelle, welche außerhalb des Wesens angeordnet ist und aus welcher sich mindestens eine erste Welle, vorzugsweise eine Schallwelle, bis zum Bewegungsvolumen ausbreitet,
- die erste Schallwelle auf einen spektralen Erreger auftrifft, welcher dadurch in Schwingung versetzt wird und dessen zum Körperteil relative Lage bekannt ist und aus welchem aufgrund der Schwingung mindestens eine zweite, ausgehende Schallwelle emittiert wird, welche sich weiterhin bis zur Schallempfängervorrichtung ausbreitet und an der Schallempfängervorrichtung bei einer festgelegten Erregungswellenlänge bzw. -frequenz isolierbar ist,
- die Schallempfängervorrichtung eine Mehrzahl oder eine variierbare Zahl 1, 2, 3, 4, 5, ...von räumlich unterscheidbaren (d.h getrennten oder zeitlich versetzbaren) Schallempfängereingängen aufweist, welche in einem bekannten Koordinatensystem derart gelagert (d.h. positioniert und orientiert) sind, dass die zweite Schallwelle jeden Schallempfängereingang oder zumindest eine maximale zahl von Schallempfängereingängen eintrifft.

Je mehr Schallempfängereingänge die zweite Schallwelle ermitteln können, desto genauer wird der Körperteil lokalisiert und desto besser ist die Messdynamik der Vorrichtung zur Lokalisation. Ist es nicht der Fall, werden die Schallempfängereingänge neu gelagert, so sie die zweite Schallwelle detektieren können.

Durch die variierbare Zahl 1, 2, 3 der Schallempfängereingänge werden einerseits ein-, zwei- oder dreidimensionale Lokalisation des Erregers in absoluter, messtechnischer Weise in dem gewünschten Koordinatensystem ermöglicht. Bei höheren Zahlen von Schallempfängereingängen oder/und Schallquellen wird die messtechnische Lokalisation genauer, mit höherer Messdynamik oder/und ggf. schneller erfolgen.

An der Schallempfängervorrichtung werden also mindestens schallbezogene, dem Bereich des Körperteils ausgehende Messsignale ermittelt, deren Laufzeiten, Frequenzen, Phasen und/oder Ausbreitungsamplituden an jedem Schallempfängereingang der Schallempfängervorrichtung je nach der Entfernung zwischen Körperteil und Schallempfängereingang variieren. Durch die Ermittlung von Eigenschaften der Messsignale wird eine Auswertung der Abstände zwischen jedem Schallempfängereingang und der Geburtstelle der zweiten Schallwelle vom Erreger durchgeführt. Dadurch dass die relativen Lagen/Entfernungen zwischen der Geburtstelle der zweiten Schallwelle und den Schallempfängereingängen bekannt gemacht worden sind und dadurch dass beide bzw. alle Schallempfängereingänge in einem bekannten, räumlichen Koördinatensystem in absoluter Weise bekannt sind, kann die genau Lage der Geburtstelle der zweiten Schalwelle in dem absoluten Koordinatensystem einfach und rasch errechnet werden. In dieser Weise ermöglicht die Erfindung eine eindeutige ein-, zwei- oder dreidimensionale Lokalisation eines innerhalb eines Bewegungsvolumens bewegbaren Körperteils (= Geburtstelle der zweiten Schallwelle) sowie eine hochschnelle Verfolgung der Bewegung des damit verbundenen Körperteils.

Dadurch, dass ein sogenanntes, zu ermitteltes Ist-Isozentrum sich mit der Bewegung des Körperteils (= des Erregers) bewegbar ist, ändert sich ein Ausgangsignal eines Schallempfängereinganges oder ändern sich gleichzeitig mehrere Ausgangsignale aus den Schallempfängereingängen. Die dadurch gebildeten und ermittelten Abweichungen des Ausgangsignals ermöglichen daher mit hoher Genauigkeit eine räumlich dynamische Verfolgung des Körperteils (= des Erregers), welche relativ zu einem Referenzpunkt (z.B. einem Soll-Isozentrum einer Bestrahlungsanlage) erfolgt. Anschließend wird angestrebt, neu entstandene Abweichungen relativ zu einem bisher lokalisierten Ist-Isozentrum dauerhaft zu ermitteln (und sie ggf. durch ein getriggertes Positionierungsmittel zu kompensieren und das Ist-Isozentrum aus dem detektieten Erreger als nächstem Soll-Isozentrum dauerhaft bekannt zu halten), so dass der Körperteil mit der Schallempfängervorrichtung immer detektierbar bleibt.

Es können selbstverständlich mehr als eine Schallquelle oder mehr als zwei räumlich getrennte Schallempfängereingänge verwendet werden. Damit wird die Genauigkeit oder/und die Geschwindigkeit der Lokalisation/Verfolgung erhöht. Dadurch können mögliche schalldämpfenden Stellen (insbesondere im Innenkörper) besser/intensiver mit Schallwellen abgedeckt werden. Die Intensität der Schallwellen wird in Sinne von minimimalen invasiven Eigenschaften derart abgestimmt, dass ein erkrankter Patient nicht gestört wird.

Die erfindungsgemäße Vorrichtung kann außerdem in Echtzeit funktionieren, so dass im dem bekannten Koordinatensystem absolute 3D-Koordinaten eines dem Erreger umfassenden Tumors (= Körperteil) abgeben werden, z.B. über 100 Aufnahme pro Sekunde. Dieses Maß ist hauptsächlich abhängig von der verwendeten Schallmesstechnologie (sowie der Rückberechnung der Messdaten in dem einzelnen Koordinatensystem). Sollten die aufgenommen Messsignale zu schwach sein bzw. zu hohe Signal-Rausch-Abstände aufweisen, können kurzzeitige Pulssignale aus der Schallquelle erzeugt werden oder/und mehrere zeitlich verschobene Messsignale an jedem Schallempfängereingang ermittelt werden, deren gezielte Messwerte statistisch addiert bzw. gemittelt werden. Damit mitteln sich auch vom Vorteil die Rausch-Werten der aufgenommenen Messwerten, allerdings auf Kosten der Aufnahmegeschwindigkeit. Unerwartete oder ungewünschte schallbezogene Messsignale können am Schallempfängereingang weg gefiltert werden, z.B. durch ein elektrisches Filter, welches einem als Schallempfängereingang vorgesehenen akustisch-elektrischen Wandler nachgeschaltet ist. Es wird ebenfalls aus Kollisions-, Schatten- oder Interferenzgründen vermieden, dass ein Komponente (Schallquelle, Schallausbreitungswege bzw. Schallempfängereingang) der erfindungsgemäßen Vorrichtung im oder entlang von einem/den Strahlgang oder -gängen einer Bestrahlungsanlage (mit z.B. einer Gamma-Strahlung für radiotherapeutische Zwecke, z.B. zur Strahlung eines Tumors oder einer Karzynom) stehen. Dies bedeutet, dass die Komponenten der erfindungsgemäßen Vorrichtung seitlich von der Gamma-Strahlung mit Hilfe von mechanischen Mitteln, wie Schaltern oder Positionierungsmodulen, positionierbar und ggf. synchron mit der Strahlungsanlage bewegbar sind, auch unter Berücksichtigung, dass der Gamma-Strahlgang seine Richtung/Position zur dreidimensionalen, räumlichen Verfolgung eines Tumors in Echtzeit ändern muss.

In Folgendem wird daher ausführlicher gezeigt, dass sehr vorteilhafte Verwendungen der Vorrichtung möglich sind. Insbesondere bei einer Verwendung, wobei der Körperteil ungesunde Zellen umfasst, beispielsweise in Form eines Tumors oder eines Karzinoms, deren dreidimensionalen Koordinaten relativ zu einem bekannten dreidimensionalen Koordinatensystem in Echtzeit ermittelt werden können. Ferner kann die Vorrichtung für eine Verwendung für sehr zahlreiche Körperteile verwendet werden, z.B. wenn sich die ungesunden, zu lokalisierten Zellen, z.B. an Augen, am Gesicht, im Lungen- oder im Brustbereich des Menschen befinden. Und schließlich können dank der Vorrichtung die dreidimensionalen Koordinaten des lokalisierten Körperteils an einer Antriebsteuerung einer Anlage zur therapeutischen Bestrahlung des Körperteils oder zur dreidimensionalen Abbildung des Körpers abgegeben oder zur Unterstützung eines therapeutischen Planungstools übermittelt bzw. verwendet werden.

Vorteilhafte Ausgestaltungen der Erfindung sind in den Unteransprüchen dargelegt.

Anschließend wird die Erfindung in einem Ausführungsbeispiel anhand der Zeichnung erläutert.

Dabei zeigen:
- FIG 1: eine erste, erfindungsgemäße Vorrichtung,
- FIG 2: eine zweite, erfindungsgemäße Vorrichtung in einer Radiotherapeutischen Bestrahlungsanlage,
- FIG 3: die zweite, erfindungsgemäße Vorrichtung in einem Liegetisch,
- FIG 4: eine dritte, erfindungsgemäße vorrichtung,
- FIG 5: eine vierte, erfindungsgemäße Vorrichtung.

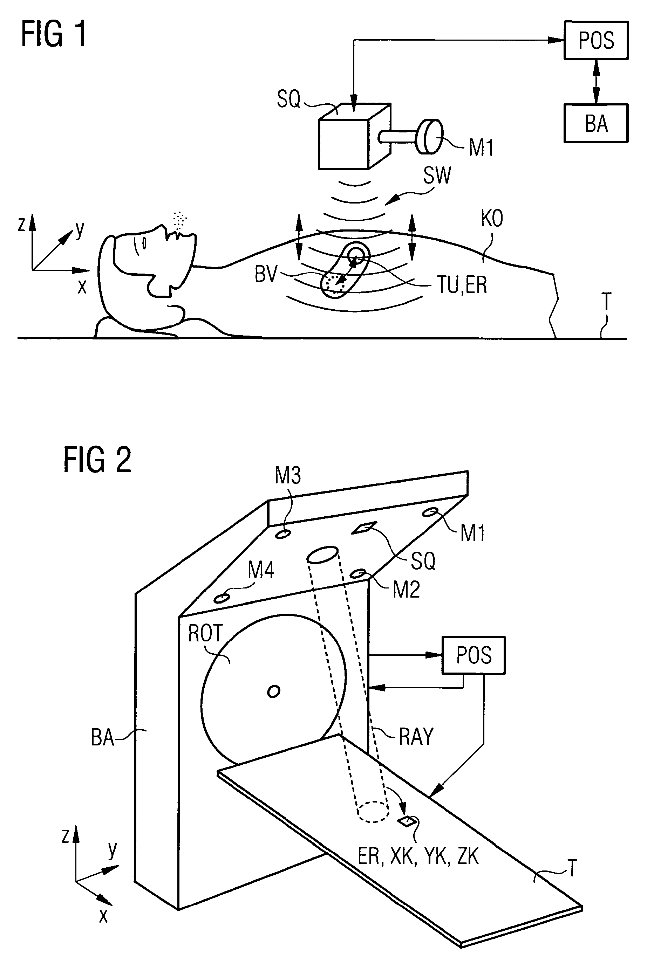

FIG 1 zeigt eine (hier nur symbolisch dargestellte) Bestrahlungsanlage BA wie einen Linearbeschleuniger zur radiotherapeutischen Behandlungen, deren mindestens ein Strahlausgang bzw. - achse einen zu vernichtenden Tumor TU (hier im Brust- oder Lungenbereich) eines auf einem Tisch T liegenden Menschen/Patienten KO zielen soll. Durch Atem oder unerwartete Bewegungen des Menschen bewegt sich unvermeidlich der Tumor TU relativ zum vorgeplanten Strahlungsziel im Körper KO und bildet daher ein sogenanntes Bewegungsvolum BV, welches alle Positionen des Tumors beinhaltet. Vor einer Bestrahlungstherapie ist es üblich, z.B. eine Computertomographie im Brust- oder Lungenbereich durchzuführen, so dass anschließend eine Neupositionierung der erfindungsgemäßen Vorrichtung gegenüber dem dadurch ermittelten Bewegungsvolumen BV des Tumors erleichtert wird. Nach der Computertomographie wird der Patient bei regelmäßigen radiotherapeutischen Behandlungen auf dem Tisch T oder einem anderen positionierbaren Tisch hingelegt, möglichst unter der gleichen Stellung als bei der ursprünglichen Computertomographie, so dass der Tumor TU als visiertes Isozentrum des Strahlgangs der Bestrahlungsanlage bleibt. Leider ist es klar, dass die Reproduzierbarkeit der regelmäßigen Positionierungen des Patienten nicht zuverlässig sein kann. Dazu verkompliziert auch der Atem die Lokalisation des Tumors. Ferner kann die Morphologie des Patienten innerhalb von Wochen geändert werden. Alle diese Faktoren addieren sich und führen zu einer ungenauen Lokalisation des gezielten Tumors.

Nun zur Beseitigung dieser Nachteilen wird in Figur 1 daher eine einfache Vorrichtung dargestellt, welche eine extern zum Patient angeordnete Schallquelle SQ aufweist, welche mindestens bis zum Tumor TU eine erste Schallwelle SW ausbreitet, wie z.B. bei einem bekannten echographischen Verfahren. Vor der tatsächlichen Lokalisation des Tumors ist ein Erreger ER in dem Tumor vorhanden, auf welchem beim Eintreffen der ersten Schallwelle eine zweite schallförmige Welle bei einer von Erreger abhängigen Frequenz oder Erregungswellenlänge emittiert wird. Ferner weist die Vorrichtung ein Mikrofon M1 auf, welches einen selektiven Empfänger der zweiten Schallwelle bildet. Am geeigneten isoliert das Mikrofon M1 die vom Erreger rücklaufende Schallwelle von anderen Schallquellen in der Umgebung des Tumors.
Nach einer geeigneten Kalibrierung der Laufzeiten/Amplituden der hin- und rücklaufenden Schallwellen ist es möglich, eine "echographische" Abstandmessung durchzuführen, welche die Entfernung zwischen der Wellenquelle SQ (oder des Mikrofons M1) und dem Erreger ER abgibt. Damit erfolgt erstmal eine eindimensionale Lokalisation des Tumors im Koordinatensystem der erfindungsgemäßen Vorrichtung.

Zur genaueren, nun bidimensionalen Lokalisation des Erregers bzw. des Tumors muss nach dem obigen Prinzip eine weitere Abstandmessung durchgeführt werden. Dafür muss die Schallempfängervorrichtung (bisheriges Mikrofon M1) messtechnisch erweitert werden. Einerseits kann einfach eine zweite Messung mit der gleichen Vorrichtung durchgeführt werden, wobei die Lage der Schallquelle oder/und des Mikrofons räumlich und in bekannter Weise geändert wird. Im Allgemeinen kann die Schallempfängervorrichtung mindestens zwei räumlich getrennte Schallempfängereingänge aufweist, welche in einem bekannten Koordinatensystem derart positioniert und orientiert sind, dass die zweite Schallwelle aus dem Erreger beide Schallempfängereingängen eintrifft. Dadurch können zwei Abstandmessungen erfolgen, welche zusammengefasst eine zweidimensionale Lokalisation des Tumors oder des Erregers ermöglichen.

Nun zur dreidimensionalen Lokalisation des Erregers weist gemäß den vorigen Ausführungsbeispielen die erfindungsgemäße Vorrichtung drei räumlich getrennte Schallempfängereingänge, z.B. mit drei Mikrofonen, welche außerhalb des Patienten in verteilter Weise, jedoch nah genug vom Erreger angeordnet sind, damit die vom Erreger rücklaufende Schallwelle mit einer Amplitude mit ausreichenden Signal-Rauschabständen an den Mikrofonen ermittelt wird.

Zur Bildung mehrerer Schallempfängereingänge die Schallempfängervorrichtung können aber die bisher beschriebenen Vorrichtungen nur einen akustisch-elektrischen Wandler, vorzugsweise einen Mikrofon gemäß Figur 1, aufweisen. Jedoch kann dabei die Zahl der Schallempfangseingänge variiert werden. Insbesondere für zwei- oder dreidimensionale Lokalisationen des gesuchten Körperteils kann der Wandler auf eine Schwenk-, Dreh- oder/und Schiebehalterung POS montiert sein oder dessen Lage und Orientierung zu dem Bewegungsvolumen mittels eines Umschaltelements modulierbar sind. Damit können zwei, drei bzw. mehrere zeitlich aufeinander folgende Messungen an dem Wandler durchgeführt werden, wobei zwischen der Messungen die räumliche Stellung des Wandlers geändert wird oder, allgemeiner formuliert, wobei der Schallempfängereingang räumlich versetzt wird. Solche Bewegungen oder Umschaltung des Schallempfängereinganges setzen voraus, dass alle neuen Positionen des Schallempfängereinganges im Koordinatensystem X, Y, Z des Tisches T ggf. durch eine Rechenaufwand bekannt sind.

Als vorteilhafte Ausführung kann der Erreger eine spezifische schallbezogenen Resonanzeigenschaft haben, um die zweite Schallwelle erzeugen zu können. Es können dabei unterschiedliche Arten von Erregern verwendet werden. Der Erreger kann z.B. eine im Körper injizierbare Substanz mit bekannten schallbezogenen Resonanzeigenschaften sein, welche sich ausschließlich und selektiv im Bereich des Körperteils (= ungesunde Zellen eines Tumors) lagert. Damit im Umfang des Tumors angeordnete Zellen keine zweite Schallwelle emittieren können, so dass lediglich der Tumorbereich in "Resonanz" gebracht wird. Der Erreger kann aber ein im Körperteil implementierbares, mechanisches Resonanzelement sein, und idealerweise in Form eines eingekapselten, akustischen Resonators umfassen. Ein solches Resonanzelement kann z.B. bei einer Biopsie in dem Tumor oder an einer zu dem Tumor bekannten, relativen Lage platziert werden. Es ist auch vorstellbar mehrere Resonanzelemente in dem Tumor oder in dessen Umfang zu platzieren, so dass aufgrund des Vielfachen von emittierenden Resonanzelementen eine dadurch additive Amplitude der zu ermittelten zweiten Schallwellen höher und daher einfacher zu bestimmen wird. Dieser Aspekt hat auch den Vorteil, eine genauere (da räumlich gemittelte) Messung der zweiten Schallwelle zu gewährleisten. Andererseits können auch die Resonanzelemente unterschiedliche Resonanzfrequenzen emittieren, so dass Messsignale an der Empfängervorrichtung z.B. durch spektrale, selektive Filterung besser getrennt werden. Dieser Aspekt kann je nach der Reichweite der Messung vermeiden, dass sich ungewünschte Interferenzen zwischen den emittierten zweiten Schallwellen bilden. Es ist ferner vorstellbar, als Erreger charakteristische Resonanzeigenschaften eines Anteils des Körperteils mit einer bekannten, erregbaren Resonanzfrequenz zu nutzen. Auch eine Tumorzelle oder, allgemeiner eine besondere erkrankte Zelle kann nach Untersuchung ihrer Eigenresonanzeigenschaft (mittels z.B. eines Schallwellenerzeugers mit variabel einstellbarer Frequenz für die erste Schallwelle und mittels eines Spektrumanalysers, zur Detektion zurückgesendeter zweiter Schallwellen) eine andere Resonanzfrequenz aufweisen als diejenige von gesunden Zellen, welche in ihrem Umfang angeordnet sind. Dabei braucht man vorteilhafterweise, keine Injizierung einer Substanz oder keine Platzierung eines Resonatorelements im Körper durchzuführen. Diese Methode ist daher für einen Patient unter Behandlung leichter zu ertragen, da eingriffslos. Im Falle einer Platzierung eines Resonatorelements an der Stelle eines zu bestrahlenden Tumors wird außerdem dieser Element Baumaterialen aufweisen, welche einen mehrwöchigen, mechanischen Funktionalitätswiderstand unter einer Belastung einer radiotherapeutischen Bestrahlung aufweisen. Es ist auch vorstellbar, dass das Resonatorelement sich mit der Zeit einfach im Körper selber weglöst, da ein solches Element aus einer einfachen Kavität besteht, welche in einem Material aus sehr unterschiedlichen Sorten (Metall, Kunststoff, etc) gebaut werden kann. Hier kann man auch aber Resonatoren mit piezo-elektrischen Elementen verwenden, welche sehr klein sind und vom Vorteil elektrisch passiv sind. Dadurch muss ein Erreger nicht mehr mittels einer Verdrahtung elektrisch versorgt werden, wie im Stand de Technik, bei welchem eine in einem Körperteil implantierten, mittels externer magnetischer Feldern zu lokalisierte Induktionsspule mit einem Draht zur Übertragung elektrischer Signale zwischen dem Körperteil und einem elektrischen Anschluss außerhalb des Körpers verwendet ist. Bei der vorliegenden Erfindung können lediglich die Resonatorelemente am Schluss einer Patientenbehandlung einfach lokalisiert und z.B. mit Hilfe eines Endoskops entfernt, wenn sie im Körper nicht lösbar sind. Die Grösse eines Resonatorelements liegt im Millimeterbereich (z.B. 0,5 bis 2 mm).

Zurück zur Bildung der Schallempfängereingänge kann auch die Schallempfängervorrichtung einfach mehrere räumlich getrennte akustisch-elektrische Wandler, vorzugsweise Mikrofone, aufweisen. Dies wird in Figur 2 erstmalig dargestellt, wobei vier Mikrofone M1, M2, M3, M4 in der Bestrahlungsanlage BA, im Umfang des Stahlsausgangs RAY zur radiotherapeutischen Behandlung angeordnet sind. Die extrinsische (= extern vom Patienten angeordnete) Schallquelle SQ ist ebenfalls zwischen den Mikrofonen M1, M2, M3, M4 angeordnet, beispielsweise neben des Strahlausgangs RAY des linearen Beschleunigers BA. Es ist auch festzustellen, dass absichtlich die Schallquelle SQ unterschiedliche Entfernungen zu den Mikrofonen hat, so dass alle Laufwege für Schallwellen aus den Paaren "Mikrofon-Schallquelle" ungleich sind. Diese Anordnung benötigt eine Vorkalibrierung der unterschiedlichen Laufwege, jedoch kann dadurch die Vorrichtung seitlich von der Gamma-Strahlung RAY angeordnet und einfach in der Bestrahlungsanlage BA installiert werden. Dadurch werden auch kürzere und höhere Laufwege der Schallwellen gebildet, welche sich für eine höhere Messdynamik je nach der Tiefe des Tumors innerhalb des Körpers besser eignen.

Die Schallempfängereingänge können auch für spektral unterschiedliche Schallwellenlängen aus dem Erreger durchlässig sein. Dies setzt voraus, dass der Erreger breitbandig emittiert oder unterschiedliche Resonanzfrequenzen erzeugen kann. Damit werden klar getrennte und daher Interferenzlose Schallsignale an die jeweilige Mikrofonen übermittelt.

Ebenfalls in Sinne einer ausreichenden Messdynamik weist die Vorrichtung einen mit der Schallquelle SQ verbundenen Schallregler der ersten Schallwelle auf, mit welchem eine Amplitude, eine Frequenz und eine Phase der ersten Schallwelle am Ausgang der Schallquelle derart eingestellt wird, dass zumindest Amplituden- oder/und Phasensignale der zweiten Schallwelle mit einem vordefinierten Signal-Rausch-Abstand an der Schallempfängervorrichtung (d.h. an möglichst allen Mikrofonen) gemessen werden. Die Mikrofone können auch zeitlich nacheinander aktiviert werden, indem unterschiedliche Einstellungen des Schallreglers bzw. der Schallquelle abrufbar sind. Dadurch wird noch mal die Messdynamik für jedes Mikrofon optimiert.

Die Schallempfängervorrichtung (d.h. die Mikrofone) kann auch akustische Filter zur spektralen Isolierung der Schallwellenlänge der zweiten Schallwelle aufweisen. Dies verhindert eine Messung einer oder mehrerer, fremder Schallquelle innerhalb sowie außerhalb des Körpers.

Die Schallempfängervorrichtung ist ferner mit einer Rechnereinheit verbunden, bei welcher mittels aufgenommener Daten, vorzugsweise an der Schallempfängervorrichtung (= an den Mikrofonen) rückgewonnener Amplitudenwerte der zweiten Schallwelle aus dem Erreger und mittels einer erfassbaren, zu einem bekannten dreidimensionalen Koordinatensystem X, Y, Z relativen Lage der Schallempfängervorrichtung dreidimensionale Koordinaten XK, YK, ZK des Körperteils in dem Koordinatensystem (X, Y, Z) in Echtzeit ermittelbar sind. Die erfassbare Lage der Schallempfängervorrichtung kann mittels eines messtechnischen Positionierungsmoduls POS ermittelt werden. Die ermittelten Koordinaten XK, YK, ZK zur Lokalisation des Erregers im Koordinatensystem X, Y, Z können weiterhin an das Positionierungsmodul POS übermittelt werden, welches anschließend die Bestrahlungsanlage BA und deren Strahlgang RAY auf den Erreger richtet. Es ist auch möglich, dass das Positionierungsmodul POS den Tisch T derart positioniert, dass der Erreger ER im Strahlgang RAY der Bestrahlungsanlage BA gehalten wird.

In Figur 2 ist ein Ausführungsbeispiel dargestellt, bei welchem die erfindungsgemäße Vorrichtung in der Bestrahlungsanlage BA integriert wurde. Jedoch, wenn diese Integration nicht möglich ist (da z.B. der Abstand zwischen der Vorrichtung und dem Patient zu groß ist und die zweite Schallwelle zu schwach wäre bzw. die erste Schallwelle zu hohe Amplituden haben sollte), kann ebenfalls die Vorrichtung neben der Bestrahlungsanlage BA geordnet werden und beispielsweise mit eigenen Positionierungsmitteln nah an dem Körper angebracht werden. Die Positionierungsmittel können jede vorstellbare, mechanische Halterung aufweisen. Hauptsache ist es, dass sie die Lage/Orientierung der erfindungsgemäßen Vorrichtung relativ zu dem absoluten Koordinatensystem liefern, so dass im gleichen Koordinatensystem der Tumor lokalisiert wird und dementsprechend die Bestrahlungsanlage BA, der Tisch T oder andere medizinische Komponente gegenüber dem Tumor (= Erreger ER) neu positioniert werden.

Damit ausreichende Signal-Rausch-Abstände bei der Aufnahme der zweiten Schallwelle an den unterschiedlichen Mikrofonen M1, M2, M3, M4 gesichert werden, obwohl der Tumor sich bewegen kann und die Dämpfung der Schallwellen variiert, können Mikrofonen an unterschiedlichen Abständen vom gezielten Körperteil angeordnet werden. Damit eignen sich zumindest ein Teil der Mikrofone (welche nah am Körperteil sind) für Signale mit schwachen Signal-Rausch-Abständen sehr gut, sowie eignen sich ein anderer Teil der Mikrofone für Signale mit hohen Signal-RauschAbständen. Das gleiche gilt für die Schallquelle SQ, welche mehrere, an unterschiedlichen Abständen vom gezielten Körperteil angeordnete Schallquellen aufweisen kann.

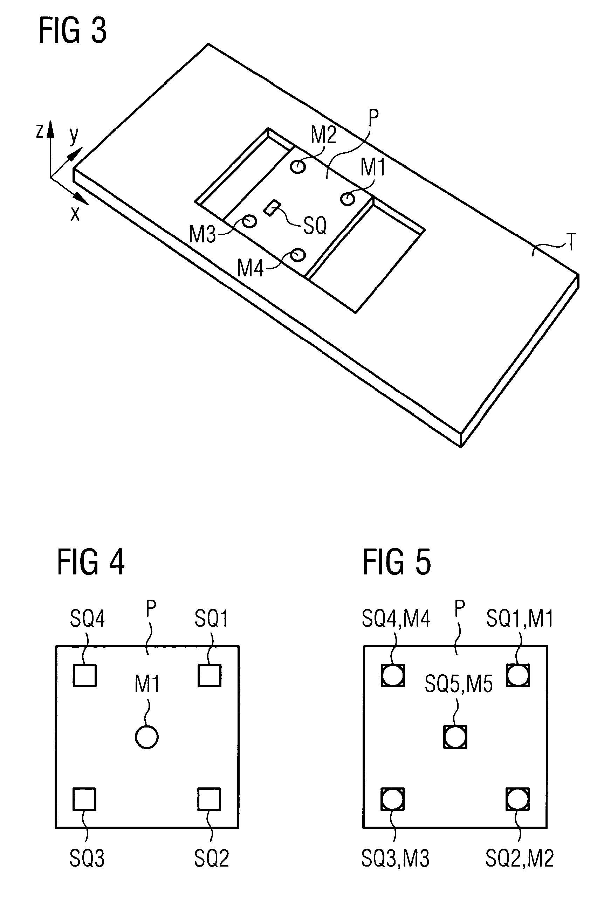

Nun zeigt Figur 3 ein weiteres Ausführungsbeispiel mit vier Mikrofonen M1, M2, M3, M4 und einer Schallquelle SQ gemäß Figur 2, jedoch werden diese Komponenten in einer Platte P angeordnet, welche zwischen dem Patient und dem Tisch T angeankert werden kann. Dies kann z.B. durch eine entlang des Tisches langförmige Ausnehmung in dem Tisch T ermöglicht werden, in welche die Platte P eingebettet wird und an einer Lage der Ausnehmung fixierbar ist, je nach dem wo der zu lokalisierte Erreger sich im Körper befindet. Damit emittiert die Schallquelle SQ die erste Schallquelle hautnah am Rücken des Körpers des liegenden Patienten und wird immer noch seitlich zu einem (hier nicht dargestellten) Strahlgang eines Linearbeschleunigers angeordnet. Damit ist sie von einer möglichen, schädlichen Strahlung geschont. Das gleiche gilt für die Mikrofonen. Die Platte P kann ggf. mit eigenen Positionierungsmitteln gekoppelt werden, welche zum Tisch T oder zum Patient relative Bewegungen der Platte ermöglichen. Dies ist aber notwendig, wenn eine zu hohe Zahl von Mikrofonen die zweite Schallwelle nicht mehr detektieren könnten (z.B. weil der Patient sich sehr viel bewegt hat).

In Figur 4 ist ein neues Ausführungsbeispiel gezeigt, welches ebenfalls einzeln oder in Kombination zu den bisherigen Ausführungsbeispielen gemäß Figur 2 (in einer Bestrahlungsanlage integriert) oder 3 (auf einer Platte P am Tisch T) verwendet sein könnte. Hier weist die erfindungsgemäße Vorrichtung anstelle einer einzelnen Schallquelle mehrere Schallquellen SQ1, SQ2, SQ3, SQ4 auf. Dies hat den Vorteil, dass der Erreger mit Schallwellen besser ausgestrahlt wird, insbesondere bei Tumoren, welche tief im Innenkörper sich befinden. Alternativerweise können auch die hier vier Schallquellen unterschiedliche Strahlungseigenschaften aufweisen, z.B. in Sinne von umfangreicheren Einstellmöglichkeiten von Amplituden, Wellenlängen, etc der erzeugten Schallwellen oder in Sinne einer umfangreicheren oder genaueren resonanzspezifischen Anpassung mit dem Erreger oder mit mehreren Erregern (ggf. mit unterschiedlichen Resonanzeigenschaften). Hier wurde nur ein Mikrofon M1 dargestellt und mittig zwischen den Schalquelle angeordnet ist. D.h. jedoch, dass die fallende radiotherapeutischen Strahlung RAY gemäß Figur 2 neben dem Mikrofon M1 gerichtet sein sollte, um zu vermeiden, dass das Mikrofon M1 messtechnisch gestört wird.

Figur 5 stellt nun ein letztes Ausführungsbeispiel gemäß Figur 4 dar, bei welchem an fünf, über die Platte P verteilten Schallquellen SQ1, SQ2, SQ3, SQ4, SQ5 fünf Mikrofonen M1, M2, M3, M4, M5 kombiniert werden. Diese Vorrichtung ermöglicht daher eine hohe Flexibilität aufgrund einer vielfältigen Einstellung von emittierbaren sowie empfangenen, schallbezogenen Eigenschaften jedes Komponenten (= jedes Mikrofons und jeder Schallquelle) sowie durch die hohe Anzahl der Komponenten höhere und genauere Messeigenschaften zur Lokalisation eines Erregers. Ggf. können eine der Schallquellen und ein der Mikrofone als ein einzelner Komponente realisiert werden, z.B. durch ein Schwingungselement nach dem Modell eines Lautsprechers.

In Figuren 3, 4, 5 ist ferner die Anordnung der Schallquellen und der Mikrofone in der ebenen Platte derart gewählt, dass bei einer Bestrahlungsanlage BA mit einer Drehachse ROT gegenüber dem Tisch T der daher um dem Tisch T drehbare Strahlgang RAY mit den Schallquellen und den Mikrofonen kontaktlos bleibt, damit die Komponenten der erfindungsgemäßen Vorrichtung messtechnisch nicht gestört werden und daher damit die gewünschte Lokalisation/verfolgung des Erregers/Körperteils immer zuverlässig erfolgt.

## Patentansprüche

1. Vorrichtung zur räumlichen Lokalisation eines bewegbaren Körperteils (TU), dessen Bewegung innerhalb eines Bewegungsvolumen (BV) an Oberflächeteilen bis auch in Innerem eines lebenden Wesens (KO) eingeschränkt ist,
**gekennzeichnet durch:**
- eine Schallempfängervorrichtung, welche außerhalb des Wesens angeordnet ist,
- eine Schallquelle (SQ), welche außerhalb des Wesens angeordnet ist und aus welcher sich mindestens eine erste Schallwelle (SW) bis zum Bewegungsvolumen ausbreitet,
- die erste Schallwelle auf einen spektralen Erreger (ER) auftrifft, welcher **dadurch** in Schwingung versetzt wird und dessen zum Körperteil relative Lage bekannt ist und aus welchem aufgrund der Schwingung mindestens eine zweite, ausgehende Schallwelle emittiert wird, welche sich weiterhin bis zur Schallempfängervorrichtung ausbreitet und an der Schallempfängervorrichtung bei einer festgelegten Erregungswellenlänge bzw. -frequenz isolierbar ist,
- die Schallempfängervorrichtung eine Mehrzahl von räumlich unterscheidbare Schallempfängereingängen (M1, M2, M3, ...) aufweist, welche in einem bekannten Koordinatensystem (X, Y, Z) derart gelagert sind, dass die zweite Schallwelle zumindest eine maximale zahl von Schallempfängereingängen eintrifft.

2. Vorrichtung nach Anspruch 1,
**dadurch gekennzeichnet, dass**
zur Bildung mehrerer Schallempfängereingänge die Schallempfängervorrichtung einen akustisch-elektrischen Wandler, vorzugsweise einen Mikrofon, aufweist, welcher auf eine Schwenk-, Dreh- oder/und Schiebehalterung montiert ist oder dessen Lage und Orientierung zu dem Bewegungsvolumen mittels eines Umschaltelements variierbar sind.

3. Vorrichtung nach Anspruch 1,
**dadurch gekennzeichnet, dass**
zur Bildung mehrerer Schallempfängereingänge die Schallempfängervorrichtung mehrere räumlich getrennte akustisch-elektrische Wandler, vorzugsweise Mikrofone, aufweist.

4. Vorrichtung nach einem der Ansprüche 2 oder 3,
**dadurch gekennzeichnet, dass**
die Schallempfängereingänge für spektral unterschiedliche Schallwellenlängen durchlässig sind.

5. Vorrichtung nach einem der voranstehenden Ansprüche,
**gekennzeichnet durch,**
einen mit der Schallquelle verbundenen Schallregler der ersten Schallwelle, mit welchem eine Amplitude, eine Frequenz und eine Phase der ersten Schallwelle am Ausgang der Schallquelle derart eingestellt wird, dass zumindest Amplituden- oder/und Phasensignale der zweiten Schallwelle mit einem vordefinierten Signal-Rausch-Abstand an der Schallempfängervorrichtung gemessen werden.

6. Vorrichtung nach einem der voranstehenden Ansprüche,
**dadurch gekennzeichnet, dass**
die Schallempfängervorrichtung akustische Filter zur spektralen Isolierung der Schallwellenlänge der zweiten Schallwelle aufweist.

7. Vorrichtung nach einem der voranstehenden Ansprüche,
**dadurch gekennzeichnet, dass**
die Schallempfängervorrichtung mit einer Rechnereinheit verbunden ist, bei welcher mittels aufgenommener Daten, vorzugsweise an der Schallempfängervorrichtung rückgewonnener Amplitudenwerte der zweiten Schallwelle und mittels einer erfassbaren, zu einem bekannten dreidimensionalen Koordinatensystem (X, Y, Z) relativen Lage der Schallempfängervorrichtung dreidimensionale Koordinaten (XK, YK, ZK) des Körperteils in dem Koordinatensystem (X, Y, Z) in Echtzeit ermittelbar sind.

8. Vorrichtung nach einem der voranstehenden Ansprüche,
**dadurch gekennzeichnet, dass**
der Erreger ein Anteil des Körperteils mit einer bekannten, erregbaren Resonanzfrequenz ist.

9. Vorrichtung nach Anspruch 8,
**dadurch gekennzeichnet, dass**
der Erreger eine im Körper injizierbare Substanz mit bekannten schallbezogenen Resonanzeigenschaften ist, welche sich ausschließlich und selektiv im Bereich des Körperteils lagert.

10. Vorrichtung nach Anspruch 8,
**dadurch gekennzeichnet, dass**
der Erreger ein im Körperteil implementierbares, mechanisches Resonanzelement ist, idealerweise in Form eines eingekapselten, akustischen Resonators umfasst.

11. Vorrichtung nach Anspruch 10,
**dadurch gekennzeichnet, dass**
das Resonatorelement Baumaterialen aufweist, welche einen mehrwöchigen, mechanischen Funktionalitätswiderstand unter einer Belastung einer radiotherapeutischen Bestrahlung auf das Resonatorelement aufweisen.

12. Vorrichtung nach einem der voranstehenden Ansprüche,
**dadurch gekennzeichnet, dass**
die Schallquelle und die Schallempfängervorrichtung seitlich von einer radiotherapeutischen Strahlung angeordnet sind.

13. Vorrichtung nach einem der voranstehenden Ansprüche,
**dadurch gekennzeichnet, dass**
die Schallquelle und die Schallempfängervorrichtung in einer Platte angeordnet sind.

14. Vorrichtung nach Anspruch 13,
**dadurch gekennzeichnet, dass**
die Platte zwischen dem Wesen und einem Tisch, auf welchem das Wesen liegt, angeordnet ist.

15. Verwendung der Vorrichtung gemäß einem der voranstehenden Ansprüche, bei welcher der Körperteil ungesunde Zellen umfasst, beispielsweise in Form eines Tumors oder eines Karzinoms, deren dreidimensionalen Koordinaten (XK, YK, ZK) relativ zu einem bekannten dreidimensionalen Koordinatensystem (X, Y, Z) in Echtzeit ermittelt werden.

16. Verwendung der Vorrichtung nach Anspruch 12, bei welcher sich die ungesunden Zellen, z.B. an Augen, am Gesicht, im Lungen- oder im Brustbereich des Menschen befinden.

17. Verwendung der Vorrichtung nach Anspruch 12 oder 13, bei welcher die dreidimensionalen Koordinaten (XK, YK, ZK) des Körperteils an einer Steuerung einer Anlage zur therapeutischen Bestrahlung des Körperteils oder zur dreidimensionalen Abbildung des Körpers abgegeben oder zur Unterstützung eines therapeutischen Planungstools verwendet werden.
